# EUROPEAN PATENT APPLICATION

(11) **EP 2 554 163 A1**
(43) Date of publication of application: **06.02.2013**
(21) Application number: 11759616.3
(22) Date of filing: 25.03.2011
(51) Int. Cl.: A61K 31/166, A61K 31/36, A61K 31/437, A61P 25/16, A61P 25/28, C07D 317/58, C07D 317/60, C07D 317/68, C07D 471/04

(54) **NEURODEGENERATIVE DISEASE THERAPEUTIC AGENT**

(30) Priority: 26.03.2010 JP 2010072820
(71) Applicant: National University Corporation Hokkaido University, Sapporo-shi Hokkaido 060-0808 (JP)
(72) Inventor: ARIGA, Hiroyoshi, Hokkaido 0600808 (JP)
(74) Representative: Flaccus, Rolf-Dieter
(86) International application number: PCT/JP2011/057458
(87) International publication number: WO 2011/118812

(57) **Abstract**

Provided is an unprecedented composition that is not a conventional symptomatic treatment, but makes possible the fundamental treatment of current neurodegenerative diseases by inhibiting oxidative-stress induced nerve-cell death. The disclosed neurodegenerative disease therapeutic agent includes a compound, or a salt of said compound, that inhibits oxidative-stress induced nerve-cell death to a high degree and is an agent used in the treatment of neurodegenerative diseases such as Parkinson's disease.

## Description

### [Technical Field]

The present invention relates to a therapeutic agent for a neurodegenerative disease.

### [Background Art]

In neurodegenerative diseases, nerve cells of a particular region of the brain die for some reasons, causing a variety of abnormalities. Although causes of onset of neurodegenerative diseases have not yet been clarified, it is assumed that oxidative stress to cells and mitochondrial dysfunction trigger the generation of protein aggregates, leading to nerve cell death. In Parkinson's disease for example, nerve cells that produce a neurotransmitter called dopamine die; therefore, dopamine precursors that are lacking as well as dopamine decomposition inhibitors are used as therapeutic agents. However, these are symptomatic treatment and cannot stop progression of nerve cell death even during treatment. Moreover, many neurodegenerative diseases do not even have a symptomatic treatment. Accordingly, there is an urgent need for development of a drug having the effect of inhibiting nerve cell death, but to date the use of such drugs has seldom been reported.

Meanwhile, DJ-1 protein is a protein having an anti-oxidative stress function, and the destruction of this function is known to be a cause of Parkinson's disease and cerebral stroke; in Non-patent Literature 1, it is reported that when DJ-1 protein is injected to a model rat of Parkinson's disease or cerebral stroke, nerve cell death and abnormal behaviors are remarkably improved. In addition, it is known that active oxygen that causes induction of oxidative stress is generated by reduced activity of complex I present in the mitochondrial inner membrane, and this reduced activity of complex I has been reported in patients with Parkinson's disease. In Non-patent Literature 2, it is reported that DJ-1 binds to this mitochondrial complex I and maintains its activity, thereby suppressing the generation of active oxygen.

In Patent Literature 1, a compound having a DJ-1 protein's suppressive effect of nerve cell death caused by oxidative stress has been described; in this document however, only the use of a compound having a backbone structure or a compound having a nucleic acid structure different from those of the present invention has been described.

### [Citation List]

### Patent Literature

Patent Literature 1: WO 2007/060886

### Non-patent Literature

Non-patent Literature 1 : Inden et al. Neurobiology of Disease (2006) 24, pp. 144-158.
Non-patent Literature 2: Hayashi et al. Biochemical and Biophysical Research Communications (2009) 390, pp. 667-672.

### [Summary of Invention]

### Problems to Be Solved by the Invention

An object of the present invention is to provide an unprecedented composition for current neurodegenerative diseases, which is not a conventional symptomatic treatment, but enables fundamental treatment by inhibiting oxidative stress-induced nerve cell death.

### Means of Solving the Problems

The present inventors have devoted themselves to the research to solve the above problem, and surprisingly found that a compound represented by Formula (1): 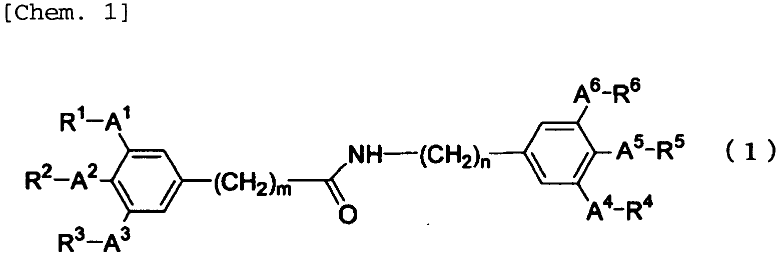 significantly suppresses oxidative stress-induced nerve cell death; after further promotion of the research, the inventors have accomplished the present invention.

Namely, the present invention relates to a therapeutic agent for a neurodegenerative disease comprising a compound represented by Formula (1): 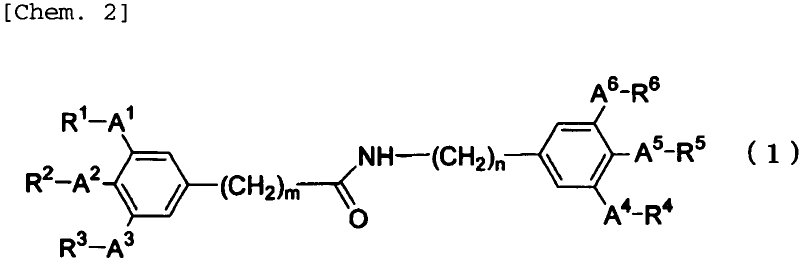 wherein
m is 0 or 1;
n is 0 or 1;
R¹ to R⁶ may be identical to or different from each other, and are a hydrogen atom, a hydroxyl group, a cyano group, a nitro group, a halogen atom, an amino group,
or
a straight or branched chain and saturated or unsaturated C₁₋₈ hydrocarbon which may have a substituent, a cycloalkyl group which may have a substituent, an aryl group which may have a substituent, a heterocyclyl group which may have a substituent;
A¹ to A⁶ are a single bond, -O-, -CO-, -COO-, -OCO-, -O-CO-O-, -S-, -SO-, -SO₂- -O-SO₂-, -NH-, -NH-COO-, -NH-SO₂-, and two adjacent -A¹-R¹ to -A⁶-R⁶ may form a ring together with the carbon atom to which they are attached;
or a pharmaceutically acceptable salt thereof.

In addition, the present invention relates to said therapeutic agent for a neurodegenerative disease, wherein m or n is 0.
Furthermore, the present invention relates to said therapeutic agent for a neurodegenerative disease, wherein m and n are 0.

In addition, the present invention relates to said therapeutic agent for a neurodegenerative disease, wherein A¹ to A⁶ are a single bond, -O-, -CO-, -COO-, -OCO-, -O-CO-O-, -NH-, or -NH-COO-.

Furthermore, the present invention relates to said therapeutic agent for a neurodegenerative disease, wherein R¹ to R⁶ may, each independently of one another, have a substituent, and are C₁₋₈ alkyl, C₂₋₈ alkenyl, C₂₋₈ alkynyl group, C₃₋₈ cycloalkyl, C₆₋₁₀ aryl, 5- to 10-membered heterocyclyl group comprising, in addition to a carbon atom, 1 to 4 heteroatoms selected from nitrogen atom, oxygen atom, and sulfur atom.

In addition, the present invention relates to said therapeutic agent for a neurodegenerative disease, wherein at least one of -A¹-R¹ to -A⁶-R⁶ is a hydrogen atom, a hydroxyl group, or the following group which may have a substituent: a straight or branched chain C₁₋₈ alkyl group, a straight or branched chain C₁₋₈ alkoxy group, a straight or branched chain C₁₋₈ alkyl-carbonyl group, a straight or branched chain C₁₋₈ alkoxy-carbonyl group, a straight or branched chain C₁₋₈ alkyl-carbonyloxy group, a straight or branched chain C₁₋₈ alkoxy-carbonyloxy group, a C₆₋₁₀ aryl group, a 5- to 10-membered heterocyclyl group (comprising, in addition to a carbon atom, 1 to 4 heteroatoms selected from nitrogen atom, oxygen atom, and sulfur atom), a C₆₋₁₀ aryloxy group, a C₆₋₁₀ aryl carbonyl, a C₆₋₁₀ aryloxy-carbonyl group, a C₆₋₁₀ aryl-carbonyloxy group, a C₆₋₁₀ aryloxy-carbonyloxy group, a 5- to 10-membered heterocyclyloxy group, a 5- to 10-membered heterocyclyl-carbonyl, a 5- to 10-membered heterocyclyloxy-carbonyl group, a 5- to 10-membered heterocyclyl-carbonyloxy group, a 5- to 10-membered heterocyclyloxy-carbonyloxy group, a C₁₋₈ alkoxycarbonylamino group.

Furthermore, the present invention relates to said therapeutic agent for a neurodegenerative disease, wherein -A²-R² and/or -A⁵-R⁵ are/is a hydrogen atom, a hydroxyl group, or the following group which may have a substituent: a straight or branched chain C₁₋₈ alkyl group, a straight or branched chain C₁₋₈ alkoxy group, a straight or branched chain C₁₋₈ alkyl-carbonyl group, a straight or branched chain C₁₋₈ alkoxy-carbonyl group, a straight or branched chain C₁₋₈ alkyl-carbonyloxy group, a straight or branched chain C₁₋₈ alkoxy-carbonyloxy group, a C₆₋₁₀ aryl group, a 5- to 10-membered heterocyclyl group (comprising, in addition to a carbon atom, 1 to 4 heteroatoms selected from nitrogen atom, oxygen atom, and sulfur atom), a C₆₋₁₀ aryloxy group, a C₆₋₁₀ aryl carbonyl, a C₆₋₁₀ aryloxy-carbonyl group, a C₆₋₁₀ aryl-carbonyloxy group, a C₆₋₁₀ aryloxy-carbonyloxy group, a 5- to 10-membered heterocyclyloxy group, a 5- to 10-membered heterocyclyl-carbonyl, a 5- to 10-membered heterocyclyloxy-carbonyl group, a 5- to 10-membered heterocyclyl-carbonyloxy group, a 5- to 10-membered heterocyclyloxy-carbonyloxy group, a C₁₋₈ alkoxycarbonylamino group.

In addition, the present invention relates to said therapeutic agent for a neurodegenerative disease, wherein -A²-R² and/or -A⁵-R⁵ are/is a straight or branched chain C₁₋₄ alkyl which may have a substituent.
Furthermore, the present invention relates to said therapeutic agent for a neurodegenerative disease, wherein -A²-R² and/or -A⁵-R⁵ are/is a monocyclic or bicyclic aryl group which may have a substituent.

In addition, the present invention relates to said therapeutic agent for a neurodegenerative disease, wherein -A²-R² and/or -A⁵-R⁵ are/is a monocyclic or bicyclic aryl group substituted by a straight or branched chain C₁₋₄ alkyl.
Furthermore, the present invention relates to said therapeutic agent for a neurodegenerative disease, wherein -A²-R² and/or -A⁵-R⁵ are/is a 5- to 10-membered monocyclic or bicyclic heterocyclyl group which may have a substituent and comprises, in addition to a carbon atom, 1 to 4 heteroatoms selected from nitrogen atom, oxygen atom, and sulfur atom.

In addition, the present invention relates to said therapeutic agent for a neurodegenerative disease, wherein -A²-R² and/or -A⁵-R⁵ are/is a 5- to 10-membered monocyclic or bicyclic heterocyclyl group comprising, in addition to a carbon atom, 1 to 4 heteroatoms selected from nitrogen atom, oxygen atom, and sulfur atom, which is substituted by a straight or branched chain C₁₋₄ alkyl.

Furthermore, the present invention relates to said therapeutic agent for a neurodegenerative disease, wherein at least one of -A¹-R¹ to -A⁶-R⁶ is a methoxy group.
In addition, the present invention relates to said therapeutic agent for a neurodegenerative disease, wherein -A¹-R¹ to -A³-R³ are a methoxy group.

Furthermore, the present invention relates to said therapeutic agent for a neurodegenerative disease, wherein the two adjacent -A¹-R¹ to -A⁶-R⁶ form a ring together with the carbon atom to which they are attached, and said ring is a ring structure represented by Formula (2):

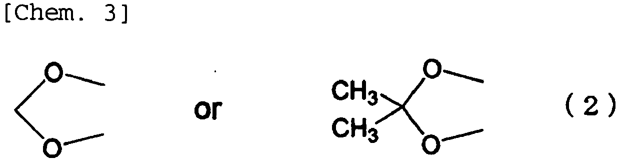

In addition, the present invention relates to said therapeutic agent for a neurodegenerative disease, wherein -A¹-R¹ and -A²-R² and/or -A⁵-R⁵ and -A⁶-R⁶ form a ring together with the carbon atom to which they are attached, and said ring is a ring structure represented by Formula (2):

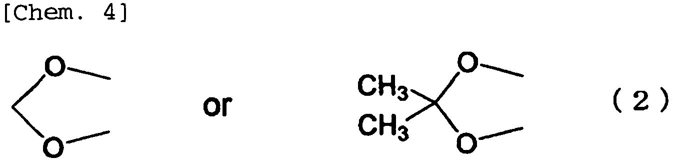

Furthermore, the present invention relates to said therapeutic agent for a neurodegenerative disease, wherein the compound represented by Formula (1) is a compound represented by Formulae (3) to (13):

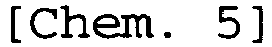

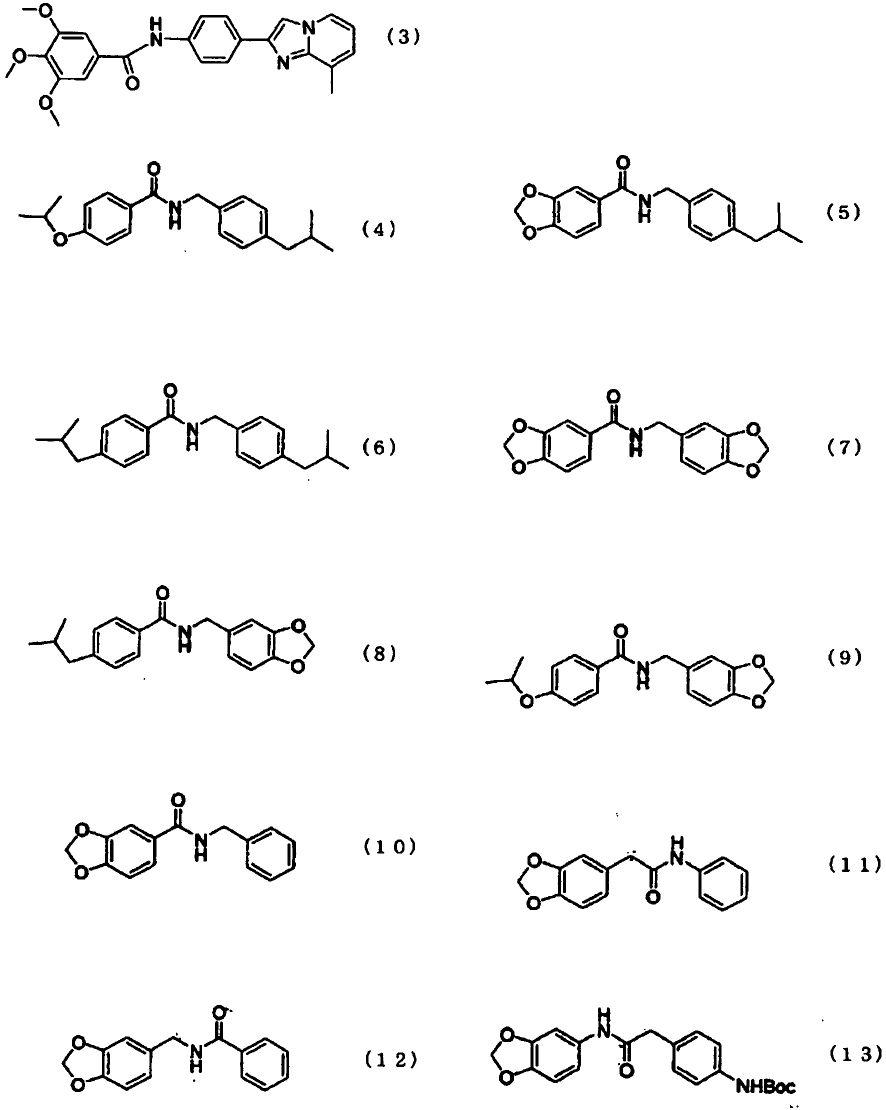

In addition, the present invention relates to said therapeutic agent for a neurodegenerative disease, wherein the compound represented by Formula (1) is a compound represented by Formula (3):

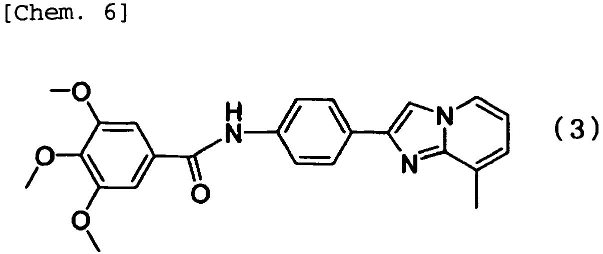

### Advantageous Effects of Invention

The present invention enables, with the above configuration, to suppress nerve cell death observed in acute neurodegenerative diseases such as cerebral stroke, and in chronic neurodegenerative diseases such as Parkinson's disease, Alzheimer's disease, Huntington's disease, ALS and apoplexy.

The function of DJ-1 protein arises from the oxidation state of 106^{th} cysteine residue (C106), and it changes depending on the level of oxidation from -SH to -SO₂H and -SO₃H. Among them, DJ-1 has an activity in the former two types (activity is the highest for -SO₂H), and it is inactive in the -SO₃H type.
In addition, DJ-1 protein acts as a positive control factor of Nrf2, a master transcription factor of a number of gene groups serving for defending oxidative stress, and DJ-1 protein performs transcriptional activation of oxidative stress-defending gene groups through Nrf2 against oxidative stress. However, DJ-1 protein as an oxidative stress-defending factor loses its function due to strong oxidation of C106 over the course of oxidative stress.

Although the action mechanism of the neurodegenerative disease therapeutic agent of the present invention has not yet been clarified, because the basic structure of the compound represented by Formula (1) well conforms to the C106 region (docking site) of DJ-1 protein, it is considered that the present agent controls the oxidation state of C106 by involvement of binding to the C106 region, thereby maintaining the activated form of DJ-1, and a pharmacological action is exerted via the anti-oxidation ability possessed by DJ-1 protein.

### [Brief Description of Drawings]

[Fig. 1] Figure 1 is a graph showing mortality rate of cells in terms of cell death caused by hydrogen peroxide.
[Fig. 2] Figure 2 is a graph showing cell death-suppressive effect of the test compound for different concentrations of hydrogen peroxide.
[Fig. 3] Figure 3 is a schematic diagram of blood vessels connected to the right side brain. In the diagram, AC represents the anterior cerebral artery, PC represents the posterior cerebral artery, MCA represents the middle cerebral artery, CCA represents the common carotid artery, and ECA represents the external carotid artery.
[Fig. 4] Figure 4 shows images of TTC (2,3,5-triphenyltetrazolium chloride) staining. In the figure, MCAO (middle cerebral artery occlusion) represents occlusion of the middle cerebral artery. "Sham" represents un-treatment, "Vehicle" represents 0.01% DMSO.
[Fig. 5] Figure 5 shows TTC-stained images of slice sections. In the figure, MCAO represents occlusion of the middle cerebral artery. "Sham" represents un-treatment, "Vehicle" represents 0.01% DMSO.
[Fig. 6] Figure 6 is a graph of plotting the area of unstained regions in the stained images, calculated for each distance from the bregma. In the figure, MCAO (middle cerebral artery occlusion) represents occlusion of the middle cerebral artery. "Sham" represents un-treatment, "Vehicle" represents 0.01% DMSO. "Infarct area" represents the area of infarct regions.
[Fig. 7] Figure 7 is a graph of calculated volume of unstained regions in the stained images. In the figure, MCAO (middle cerebral artery occlusion) represents occlusion of the middle cerebral artery. "Sham" represents un-treatment, "Vehicle" represents 0.01% DMSO. "Infarct volume" represents the volume of infarct regions.

[Fig. 8] Figure 8 is a graph showing survival rate of cells in terms of cell death caused by 24-h treatment with 50 µM 6-OHDA (6-hydroxydopamine).
[Fig. 9 Figure 9 is a graph showing survival rate of cells in terms of cell death caused by 1-h treatment with 125 µM 6-OHDA.
[Fig. 10] Figure 10 is a graph showing results of measurement of active oxygen species by a fluorescence spectrophotometer.
[Fig. 11] Figure 11 shows immunostaining of cell cultures of primary nerve cells of the ventral mesencephalon of a mouse. Staining with anti-NeuN antibody represented by α-NeuN is observed in green color. Staining with anti-TH antibody represented by α-TH is observed in red color. Straining with DAPI is observed in blue color. "Merge" represents integration of the stained images, and is observed in yellow-white color.
[Fig. 12] Figure 12 is a graph showing survival rate of cells in terms of cell death caused by hydrogen peroxide.
[Fig. 13] Figure 13 is a diagram showing ESR spectra.
[Fig. 14] Figure 14 is a graph showing quantification of in-vitro hydroxyl radical production. Each value represents mean +/standard error of 8 measurements relative to the control as 100%.
[Fig. 15] Figure 15 is a graph showing the total number of rotations in mice up to 7 days after administration.
[Fig. 16] Figure 16 is a graph showing time-course changes in the number of rotations.

### [Description of Embodiments]

Hereinafter, the present invention is described in detail.
In the compound comprised in the neurodegenerative disease therapeutic agent of the present invention, in general formula (1) : 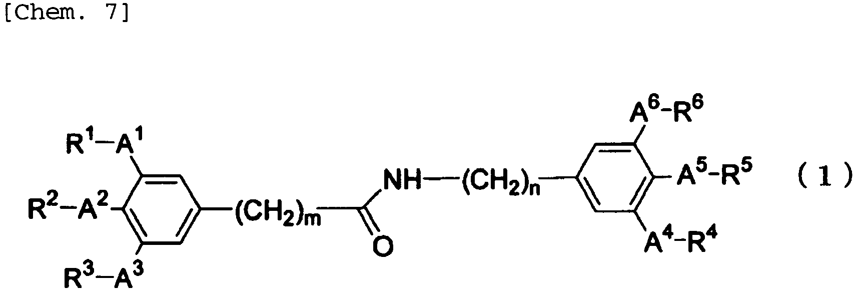 m is 0 or 1, and preferably m is 0. n is 0 or 1, and preferably n is 0.

R¹ to R⁶ in general formula (1) of the present invention may be identical to or different from each other, and are a hydrogen atom, a hydroxyl group, a cyano group, a nitro group, a halogen atom, an amino group, or a straight or branched chain and saturated or unsaturated C₁₋₈ hydrocarbon having 1-8 carbon atoms which may have a substituent, a cycloalkyl group which may have a substituent, an aryl group which may have a substituent, a heterocyclyl group which may have a substituent.

In general formula (1), examples of the halogen atom as R¹ to R⁶ include, but are not limited to, fluorine, chlorine, bromine and iodine.

In general formula (1), examples of the amino group as R¹ to R⁶ include, but are not limited to, amino group and di-C₁₋₈ alkylamino group.

In general formula (1), examples of the straight or branched chain and saturated or unsaturated hydrocarbon which may have a substituent as R¹ to R⁶ include, but are not limited to, straight or branched chain C₁₋₈ alkyl group, C₂₋₈ alkenyl group and C₂₋₈ alkynyl group.

The straight or branched chain C₁₋₈ alkyl group represents, but is not limited to, a straight or branched chain alkyl group having 1 to 8 carbon atoms such as methyl, ethyl, propyl, isopropyl, butyl, isobutyl, sec-butyl, tert-butyl, pentyl, hexyl, heptyl, and octyl. It is preferably a straight or branched chain alkyl group having 1 to 4 carbon atoms.

The straight or branched chain C₂-₈ alkenyl group represents, but is not limited to, a straight or branched chain alkenyl group having 2 to 8 carbon atoms such as vinyl, allyl, propenyl, butenyl, pentenyl, hexenyl, heptenyl, and octenyl. It is preferably a straight or branched chain alkenyl group having 2 to 4 carbon atoms.

The straight or branched chain C₂₋₈ alkynyl group represents, but is not limited to, a straight or branched chain alkynyl group having 2 to 8 carbon atoms such as ethynyl, propynyl, butynyl, pentynyl, hexynyl, heptynyl, and octynyl. It is preferably a straight or branched chain alkynyl group having 2 to 4 carbon atoms.

In general formula (1), examples of the cycloalkyl group as R¹ to R⁶ include, but are not limited to, for example, cyclopropyl, cyclobutyl, cyclopentyl, cyclohexyl, cycloheptyl, and cyclooctyl.

Examples of the aryl group which may have a substituent as R¹ to R⁶ in general formula (1) include, but are not limited to, for example, a monocyclic or bicyclic aryl group having 6 to 10 carbon atoms, such as phenyl, naphthyl (including those with a part or the whole ring of the aryl group is saturated).

The heterocyclyl group which may have a substituent as R¹ to R⁶ in general formula (1) represents a ring structure comprising one or more heteroatoms in addition to a carbon atom, and such ring structure encompasses those having aromatic property and those without aromatic property. Examples include a 5- to 10-membered monocyclic or bicyclic heterocyclyl group having 1 to 4 heteroatoms selected from nitrogen atom, oxygen atom, and sulfur atom, in addition to a carbon atom, and the selected heteroatoms may be the same or different. Examples include, but are not limited to, thiophene, furan, pyran, pyridine, pyrrole, pyrazine, azepine, azocine, azonine, azecine, oxazole, thiazole, pyrimidine, pyridazine, triazine, triazole, tetrazole, imidazole, pyrazole, morpholine, thiomorpholine, piperidine, piperazine, quinoline, isoquinoline, indole, isoindole, quinoxaline, phthalazine, quinolizine, quinazoline, naphthyridine, chromene, benzofuran, benzothiophene, pyrrolidine, pyridine, indolizine, indazole, and purine. Preferably, it is a monocyclic or bicyclic heterocyclyl group comprising a nitrogen atom, and particularly preferably, a heterocyclyl group represented by the following structures:

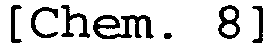

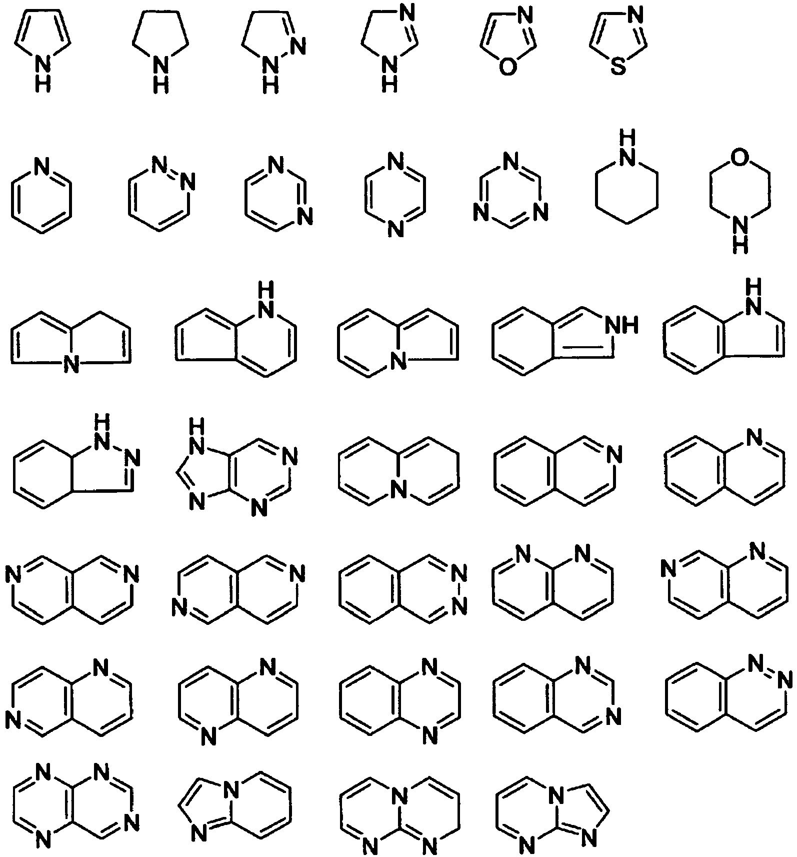

R¹ to R⁶ in general formula (1) are, preferably, each independently of one another, hydrogen atom, C₁₋₈ alkyl, C₂₋₈ alkenyl, C₂₋₈ alkynyl group, C₃₋₈ cycloalkyl, C₆₋₁₀ aryl, 5- to 10-membered heterocyclyl group comprising, in addition to a carbon atom, 1 to 4 heteroatoms selected from nitrogen atom, oxygen atom, and sulfur atom.

The above R¹ to R⁶ may have a substituent, and examples of such substituent include, but are not limited to, for example, a hydroxyl group, a cyano group, a nitro group, a halogen atom (for example, fluorine, chlorine, bromine, etc.), an amino group, and the following straight or branched chain groups: C₁₋₈ alkyl group, C₁₋₈ alkoxy group, C₁₋₈ alkyl-carbonyl group, C₁₋₈ alkoxy-carbonyl group, C₁₋₈ alkyl-carboxyl group, C₁₋₈ alkoxy-carboxyl group, or a monocyclic or bicyclic C₆₋₁₀ aryl group, a 5- to 10-membered monocyclic or bicyclic heterocyclyl group (which comprises, in addition to a carbon atom, 1 to 4 heteroatoms selected from nitrogen atom, oxygen atom, and sulfur atom), a monocyclic or bicyclic C₆₋₁₀ aryloxy group, a monocyclic or bicyclic C₆₋₁₀ aryl-carbonyl, a monocyclic or bicyclic C₆₋₁₀ aryloxy-carbonyl group, a monocyclic or bicyclic C₆₋₁₀ aryl-carboxyl group, a monocyclic or bicyclic C₆₋₁₀ aryloxy-carboxyl group, a 5- to 10-membered monocyclic or bicyclic heterocyclyloxy group, a 5- to 10-membered monocyclic or bicyclic heterocyclyl-carbonyl, a 5- to 10-membered monocyclic or bicyclic heterocyclyloxy-carbonyl group, a 5- to 10-membered monocyclic or bicyclic heterocyclyl-carboxyl group, a 5- to 10-membered monocyclic or bicyclic heterocyclyloxy-carboxyl group, etc. R¹ to R⁶ may have one or more above substituents at a replaceable position, and when the number of substituents is 2 or more, each substituent may be identical or different.

Accordingly, examples of R¹ to R⁶ may include, but are not limited to, the structures, etc. listed below when the following group: 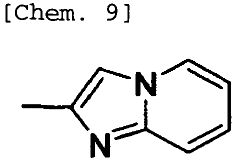 is a backbone;

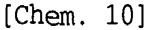

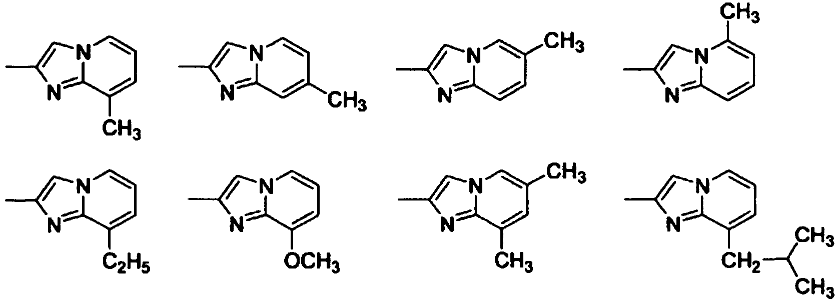

A¹ to A⁶ in general formula (1) are a single bond, -O-, -CO-, -COO-, -OCO-, -OCO-O-, -S-, -SO-, -SO₂-, -O-SO₂-, -NH-, -NH-COO- or -NH-SO₂-. Preferably, A¹ to A⁶ are a single bond, -O-, -CO-, -COO-, -OCO-, -OCO-O-, -NH- or -NH-COO-.

When A¹ to A⁶ in general formula (1) are a single bond, typical examples of -A¹-R¹ to -A⁶-R⁶ include a hydrogen atom, a hydroxyl group, a cyano group, a nitro group, a halogen atom, an amino group, a straight or branched chain C₁₋₈ alkyl which may be substituted, and preferably, a straight or branched chain C₁₋₄ alkyl, a cycloalkyl group, an aryl group, a heterocyclyl group.

When A¹ to A⁶ are -O-, typical examples of -A¹-R¹ to -A⁶-R⁶ include, but are not limited to, a straight or branched chain C₁₋₈ alkoxy group such as methoxy, ethoxy, propoxy, and butoxy, a monocyclic or bicyclic C₆₋₁₀ aryloxy group such as phenoxy and naphthyloxy, and a 5- to 10-membered monocyclic or bicyclic heterocyclyloxy group comprising, in addition to a carbon atom, 1 to 4 heteroatoms selected from nitrogen atom, oxygen atom, and sulfur atom.

When A¹ to A⁶ are-CO-, typical examples of -A¹-R¹ to -A⁶-R⁶ include, but are not limited to, a straight or branched chain C₁₋₈ alkyl-carbonyl group such as acetyl, propionyl, butyryl, and isobutyryl, a monocyclic or bicyclic C₆₋₁₀ aryl-carbonyl group such as phenylcarbonyl group and naphthylcarbonyl, and a 5- to 10-membered monocyclic or bicyclic heterocyclyl-carbonyl group comprising, in addition to a carbon atom, 1 to 4 heteroatoms selected from nitrogen atom, oxygen atom, and sulfur atom.

When A¹ to A⁶ are -COO-, typical examples of -A¹-R¹ to -A⁶-R⁶ include, but are not limited to, a carboxyl group, or a straight or branched chain C₁₋₈ alkoxy-carbonyl group such as methoxycarbonyl, ethoxycarbonyl, propoxycarbonyl, and butoxycarbonyl, a monocyclic or bicyclic C₆₋₁₀ aryloxy-carbonyl group such as phenoxycarbonyl and naphthyloxycarbonyl, and a 5- to 10-membered monocyclic or bicyclic heterocyclyloxy-carbonyl group comprising, in addition to a carbon atom, 1 to 4 heteroatoms selected from nitrogen atom, oxygen atom, and sulfur atom.

When A¹ to A⁶ are -OCO-, typical examples of -A¹-R¹ to -A⁶-R⁶ include, but are not limited to, a straight or branched chain C₁₋₈ alkyl-carbonyloxy group such as methylcarbonyloxy, ethylcarbonyloxy, and propylcarbonyloxy, a monocyclic or bicyclic C₆₋₁₀ arylcarbonyloxy group such as phenylcarbonyloxy group and naphthylcarbonyloxy group, and a 5- to 10-membered monocyclic or bicyclic heterocyclylcarbonyloxy group comprising, in addition to a carbon atom, 1 to 4 heteroatoms selected from nitrogen atom, oxygen atom, and sulfur atom.

When A¹ to A⁶ are -O-CO-O-, typical examples of -A¹-R¹ to -A⁶-R⁶ include, but are not limited to, a straight or branched chain C₁₋₈ alkoxy-carbonyloxy group such as methoxycarbonyloxy, ethoxycarbonyloxy, propoxycarbonyloxy, and butoxycarbonyloxy, a monocyclic or bicyclic C₆₋₁₀ aryloxycarbonyloxy group such as phenoxycarbonyloxy and naphthyloxycarbonyloxy, and a 5- to 10-membered monocyclic or bicyclic heterocyclylcarbonyloxy group comprising, in addition to a carbon atom, 1 to 4 heteroatoms selected from nitrogen atom, oxygen atom, and sulfur atom.

When A¹ to A⁶ are -S-, typical examples of -A¹-R¹ to -A⁶-R⁶ include, but are not limited to, a C₁₋₈ alkylthio group such as methylthio, ethylthio, propylthio, andbutylthio, a monocyclic or bicyclic C₆₋₁₀ arylthio group such as phenylthio and naphthylthio, and a 5- to 10-membered monocyclic or bicyclic heterocyclylthio group comprising, in addition to a carbon atom, 1 to 4 heteroatoms selected from nitrogen atom, oxygen atom, and sulfur atom.

When A¹ to A⁶ are -SO-, typical examples of -A¹-R¹ to -A⁶-R^{6.} include, but are not limited to, a C₁₋₈ alkylsulfinyl group such as methylsulfinyl, ethylsulfinyl, propylsulfinyl, and butylsulfinyl, a monocyclic or bicyclic C₆₋₁₀ arylsulfinyl group such as phenylsulfinyl and naphthylsulfinyl, and a 5- to 10-membered monocyclic or bicyclic heterocyclylsulfinyl group comprising, in addition to a carbon atom, 1 to 4 heteroatoms selected from nitrogen atom, oxygen atom, and sulfur atom.

When A¹ to A⁶ are -SO₂- typical examples of -A¹-R¹ to -A⁶-R⁶ include, but are not limited to, a straight or branched chain C₁₋₈ alkylsulfonyl group such as methylsulfonyl, ethylsulfonyl, propylsulfonyl, and butylsulfonyl, a monocyclic or bicyclic C₆₋₁₀ arylsulfonyl group such as phenylsulfonyl and naphthylsulfonyl, and a 5- to 10-membered monocyclic or bicyclic heterocyclylsulfonyl group comprising, in addition to a carbon atom, 1 to 4 heteroatoms selected from nitrogen atom, oxygen atom, and sulfur atom.

When A¹ to A⁶ in general formula (1) are -O-SO₂-, typical examples of -A¹-R¹ to -A⁶-R⁶ include, but are not limited to, a straight or branched chain C₁₋₈ alkylsulfonyloxy group such as methylsulfonyloxy, ethylsulfonyloxy, propylsulfonyloxy, and butylsulfonyloxy, a C₆₋₁₀ arylsulfonyloxy group such asphenylsulfonyloxy and naphthylsulfonyloxy, and a 5- to 10-membered monocyclic or bicyclic heterocyclylsulfonyloxy group comprising, in addition to a carbon atom, 1 to 4 heteroatoms selected from nitrogen atom, oxygen atom, and sulfur atom.

When A¹ to A⁶ are -NH-, typical examples of -A¹-R¹ to -A⁶-R⁶ include, but are not limited to, a C₁₋₈ alkylamino group such as methylamino, ethylamino, propylamino, and butylamino, a C₆₋₁₀ arylamino group such as phenylamino and naphthylamino, and a 5-to 10-membered monocyclic or bicyclic heterocyclylamino group comprising, in addition to a carbon atom, 1 to 4 heteroatoms selected from nitrogen atom, oxygen atom, and sulfur atom.

When A¹ to A⁶ are -NH-COO-, typical examples of -A¹-R¹ to -A⁶-R⁶ include, but are not limited to, a straight or branched chain C₁₋₈ alkoxycarbonylamino group such as tert-butoxycarbonylamino group ( -NHBoc), a monocyclic or bicyclic C₆₋₁₀ aryloxy-carbonylamino group such as phenoxycarbonylamino group and naphthyloxycarbonylamino group, and a 5- to 10-membered monocyclic or bicyclic heterocyclyloxycarbonylamino group comprising, in addition to a carbon atom, 1 to 4 heteroatoms selected from nitrogen atom, oxygen atom, and sulfur atom.

When A¹ to A⁶ are -SO₂-NH-, typical examples of -A¹-R¹ to -A⁶-R⁶ include, but are not limited to, a straight or branched chain C₁₋₈ alkylsulfamoyl group such as sulfamoyl, methylsulfamoyl, ethylsulfamoyl, propylsulfamoyl, and butylsulfamoyl, amonocyclic or bicyclic C₆₋₁₀ arylsulfamoyl group such as phenylsulfamoyl and naphthylsulfamoyl, and a 5- to 10-membered monocyclic or bicyclic heterocyclylsulfamoyl group comprising, in addition to a carbon atom, 1 to 4 heteroatoms selected from nitrogen atom, oxygen atom, and sulfur atom.

-A¹-R¹ to -A⁶-R⁶ are, preferably each independently of one another, a hydrogen atom, a hydroxyl group, a straight or branched chain C₁₋₈ alkyl group, a straight or branched chain C₁₋₈ alkoxy group, a straight or branched chain C₁₋₈ alkyl-carbonyl group, a straight or branched chain C₁₋₈ alkoxy-carbonyl group, a straight or branched chain C₁₋₈ alkyl-carbonyloxy group, a straight or branched chain C₁₋₈ alkoxy-carbonyloxy group, a monocyclic or bicyclic C₆₋₁₀ aryl group, a 5- to 10-membered monocyclic or bicyclic heterocyclyl group (that comprises, in addition to a carbon atom, 1 to 4 heteroatoms selected from nitrogen atom, oxygen atom, and sulfur atom), a monocyclic or bicyclic C₆₋₁₀ aryloxy group, a monocyclic or bicyclic C₆₋₁₀ aryl-carbonyl, a monocyclic or bicyclic C₆₋₁₀ aryloxy-carbonyl group, a monocyclic or bicyclic C₆₋₁₀ aryl-carbonyloxy group, a monocyclic or bicyclic C₆₋₁₀ aryloxy-carbonyloxy group, a 5- to 10-membered monocyclic or bicyclic heterocyclyloxy group, a 5- to 10-membered monocyclic or bicyclic heterocyclyl-carbonyl, a 5- to 10-membered monocyclic or bicyclic heterocyclyloxy-carbonyl group, a 5- to 10-memberedmonocyclic or bicyclic heterocyclyl-carbonyloxy group, a 5- to 10-membered monocyclic or bicyclic heterocyclyloxy-carbonyloxy group, a C₁₋₈ alkoxycarbonylamino group.

The above -A¹-R¹ to -A⁶-R⁶ may have a substituent. Accordingly, -A¹-R¹ to -A⁶-R⁶ may be, but are not limited to, alkoxyalkyl such as methoxymethyl, methylphenyl, or a monocyclic or bicyclic heterocyclyl group comprising nitrogen atoms and having a substituent, such as the following structure:

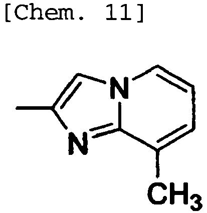

In one preferred embodiment of the present invention, -A²-R² and/or -A⁵-R⁵ in general formula (1) are not a hydrogen atom, but A¹-R¹, -A³-R³, -A⁴-R⁴, -A⁵-R⁵, -A⁶-R⁶ are a hydrogen atom. In this case, -A²-R² and/or -A⁵-R⁵ may be straight or branched chain C₁₋₄ alkyl, straight or branched chain C₁₋₄ alkoxy, monocyclic or bicyclic aryl, or a 5- to 10-membered monocyclic or bicyclic heterocyclyl group comprising, in addition to a carbon atom, 1 to 4 heteroatoms selected from nitrogen atom, oxygen atom, and sulfur atom, and R² and/or R⁵ may be mono- or di-substituted by straight or branched chain C₁₋₄ alkyl.

In one preferred embodiment of the present invention, -A¹-R¹ to -A³-R³ in general formula (1) are either a hydrogen atom, straight or branched chain C₁₋₄ alkyl, or straight or branched chain C₁₋₄ alkoxy, and any of -A⁴-R⁴ to -A⁶-R⁶ are monocyclic or bicyclic aryl, or a 5- to 10-membered monocyclic or bicyclic heterocyclyl group comprising, in addition to a carbon atom, 1 to 4 heteroatoms selected from nitrogen atom, oxygen atom, and sulfur atom. Here, it is preferable that any one of -A⁴-R⁴ to -A⁶-R⁶ is aryl or heterocyclyl group. In addition, R⁴ to R⁶ may be mono- or di-substituted by straight or branched chain C₁₋₄ alkyl.

In one embodiment of the present invention, at least one of -A¹-R¹ to -A⁶-R⁶ in general formula (1) is a C₁₋₈ alkoxy group, and preferably a C₁₋₄ alkoxy group, and particularly preferably a methoxy group. All of -A¹-R¹ to -A³-R³ may be a methoxy group. In this case, it is preferable that all of -A¹-R¹ to -A³-R³ are a methoxy group, -A⁴-R⁴ and -A⁶-R⁶ are hydrogen, and A⁵-R⁵ is a heterocyclyl group which may have a substituent.

Two adjacent groups in -A¹-R¹ to -A⁶-R⁶ in general formula (1), preferably -A¹-R¹ and -A²-R², and/or A⁵-R⁵ and A⁵-R⁵, may form a ring together with the carbon atom to which they are attached. Examples of said ring include, but are not limited to, cycloalkyl such as cyclopropyl, cyclobutyl, cyclopentyl, cyclohexyl, cycloheptyl, cyclooctyl, and a hetero ring represented by, for example, formula (2):

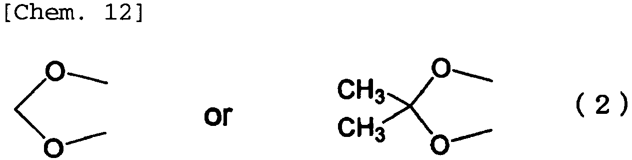

The compounds of the following formulae (3) to (13) are preferred; however, the present invention is not limited thereto.

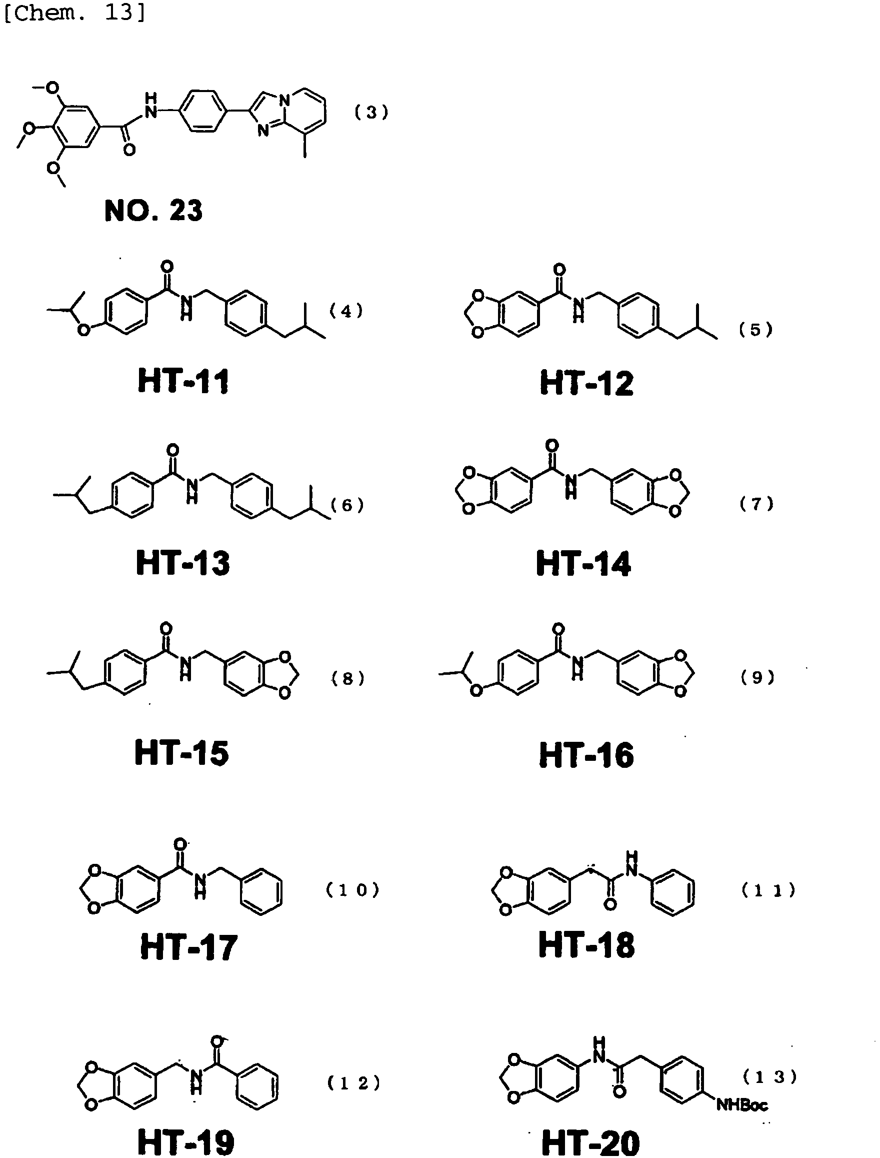

A particularly preferred compound is No. 23 represented by the following formula (3): 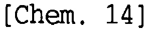 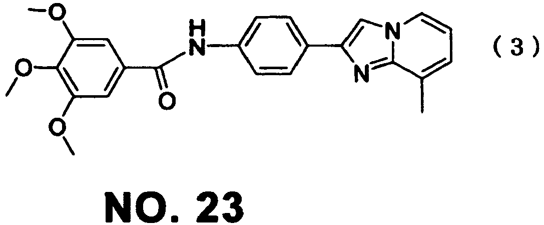 No. 23 has a particularly high cell death suppressive strength. Although the reason for this is not clear, possibly it derives from the predicted steric structure of the molecule. Compared to other compounds, the structure of No. 23 is speculated to cover the active site of C106 region to an appropriate extent, and due to this steric structure, high level of oxidation of C106 region is prevented, and suitable oxidation state can be maintained.

The compound represented by general formula (1) can be converted by publicly-known method to form a pharmaceutically acceptable salt. For example, the compounds having, in its group, carboxyl group, sulfo group or amino group include a salt thereof.

Examples of the salt include alkali metal salts, alkaline earth metal salts, ammonium salts, amine salts, acid addition salts, etc.
Suitable examples thereof include a salt of alkali metals (e. g. , potassium and sodium), a salt of alkaline earth metals (e. g. , calcium and magnesium), a salt of ammonium (e.g., tetramethyl ammonium), a salt of organic amines (e.g., triethylamine, methylamine, dimethylamine, cyclopentylamine, benzylamine, phenethylamine, piperidine, monoethanolamine, diethanolamine, tris (hydroxymethyl) aminomethane, lysine, arginine, N-methyl-D-glucamine).

Suitable acid addition salts include, for example, inorganic acid salts such as hydrochloride, hydrobromide, hydroiodide, sulfate, phosphate, and nitrate, or organic acid salts such as acetate, lactate, tartrate , benzoate, citrate, methanesulfonate, ethanesulfonate, benzenesulfonate, toluenesulfonate, isethionate, glucuronate, and gluconate.

In addition, a compound of general formula (1) and a pharmaceutically acceptable salt thereof may be converted to a solvate by publicly known method. Examples of appropriate solvate include a solvate with water and alcoholic solvents (e.g., ethanol, methanol, benzyl alcohol), ether solvents (e.g., diethyl ether, ethyl methyl ether), ester solvents (e.g., ethyl acetate, butyl acetate), benzene solvents (e.g., benzene, toluene, xylene), halogen solvents (e.g., chloroform, dichloromethane).
The compound (1) of the present invention may be a hydrate or a non-hydrate.

The concentration of the compound of the present invention comprised in the neurodegenerative disease therapeutic agent of the present invention is not particularly limited. The neurodegenerative disease therapeutic agent of the present invention may comprise any component other than the above compound, as long as the component does not undermine the effect of the present invention. The administration method of the neurodegenerative disease therapeutic agent of the present invention is not particularly limited (oral administration, injection administration, etc.), and its dosage form is not particularly limited (powder, tablet, injection solution, etc.).

### Examples

Hereinafter, the present invention is described in further detail with reference to examples; however, the present invention is not limited to these examples.

### [Example 1] Cell death-suppressive effect of DJ-1 bound compound in cultured cells

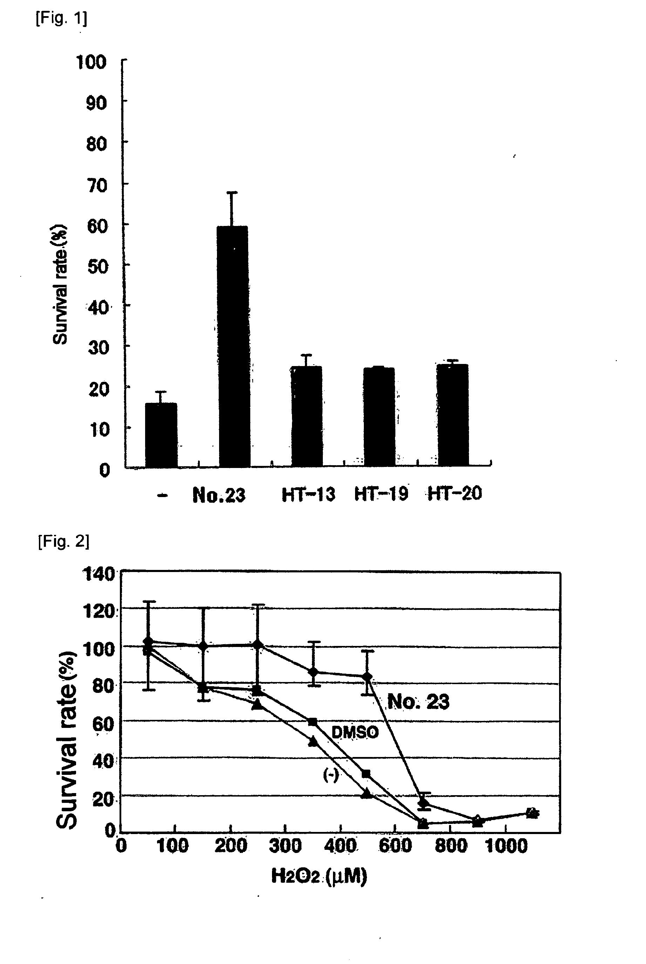

To a culture solution of human dopamine-producing cell SH-SY5Y, the following DJ-1 bound compounds dissolved in 0.01% DMSO: No. 23 represented by formula (3) (ENAMINE Ltd.),
HT13 represented by formula (6) (provided from Mr. Toshio Honda, Hoshi University),
HT19 represented by formula (12) (provided from Mr. Toshio Honda, Hoshi University), and
HT20 represented by formula (13) (provided from Mr. Toshio Honda, Hoshi University)
at 1 µM each were administered for 24 h, then 250 µM H₂O₂ was added and treated for 2 h, and the ratio of the cell death was investigated by MTT assay. As control, a control (-) without addition of a compound was used. It was demonstrated that compared to the control, cell death in the samples was suppressed by the DJ-1 bound compounds, in particular by No. 23 (Fig. 1).

### [Example 2] Cell death-suppressive effect of DJ-1 bound compound in cultured cells for different concentrations of hydrogen peroxide

To a culture solution of human dopamine-producing cell SH-SY5Y, 1 µM DJ-1 bound compound (No. 23) dissolved in 0.01% DMSO was administered for 24 h, then various concentrations of H₂O₂ were added and treated for 2 h, and the ratio of cell death was investigated by MTT assay. For comparison, a control (-) without addition of the DJ-1 bound compound and a control (DMSO) with addition of only 0.01% DMSO were used and subjected to the same method. While the both controls showed an increase of cell death from 150 µM, the DJ-1 bound compound (No. 23) significantly suppressed cell death up to the high H₂O₂ concentration of 500 µM (Fig. 2).

### [Example 3] Cell death-suppressive effect of DJ-1 bound compound in cerebral stroke model rat

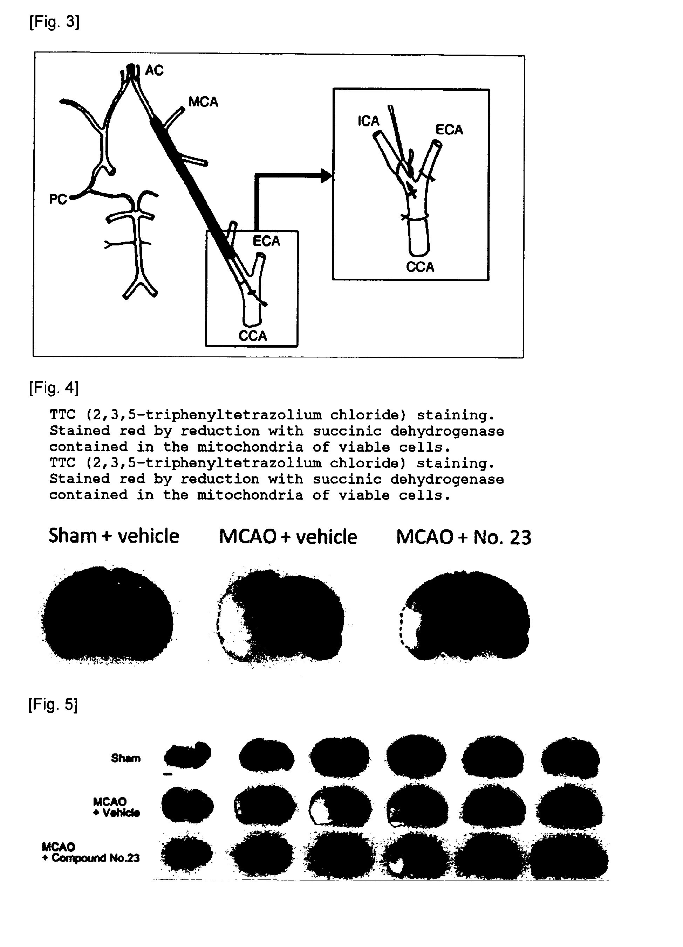

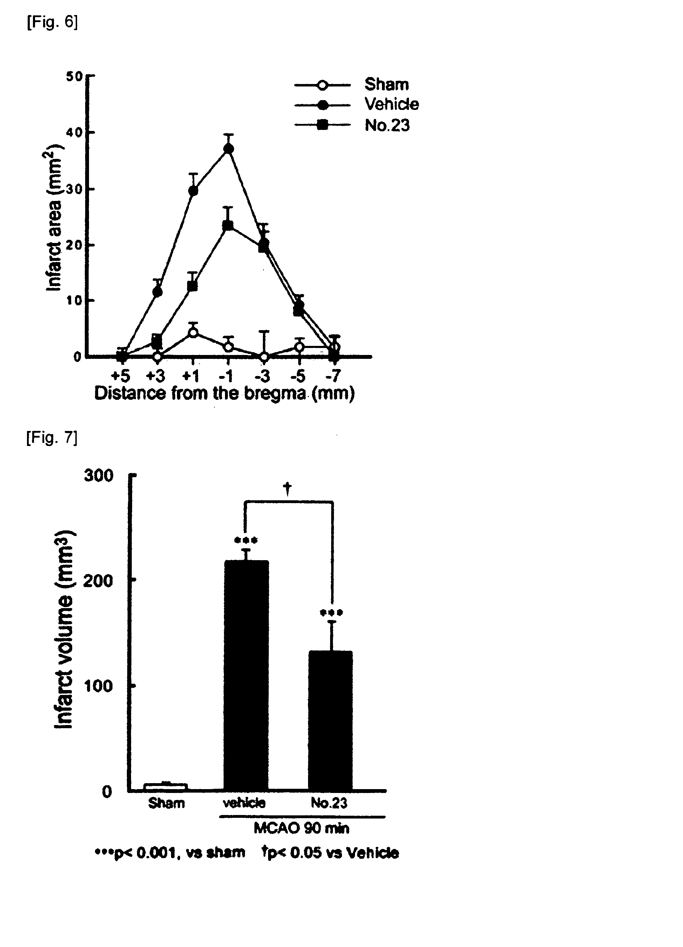

To the left brain of a rat (left striatum: 1-mm anterior, 4-mm left-lateral and 5-mm ventral from the bregma), the DJ-1 bound compound (No. 23) dissolved in 0.01% DMSO was injected, and 30 min after, the blood vessel connecting to the right lateral brain was occluded for 90 min (Fig. 3). After 24-h re-perfusion, a brain section slice was made and TTC-stained. For comparison, a control (-) without addition of the DJ-1 bound compound and a control (DMSO) with addition of only 0.01% DMSO were used and subjected to the same method. Results are shown in Fig. 4. Occluded layers where cell death occurs are not stained. Figure 5 shows a diagram of staining of the brain at corresponding distance from the bregma, and Fig. 6 shows the area of un-stained regions at different distances from the bregma. Figure 7 shows results of calculation of total volume of unstained regions. Due to the administration of No. 23, the area of the TTC-stained regions decreases, demonstrating that No. 23 significantly suppresses cell death.

### [Example 4] Effect of suppression of oxidative stress-induced nerve cell death by DJ-1 bound compound

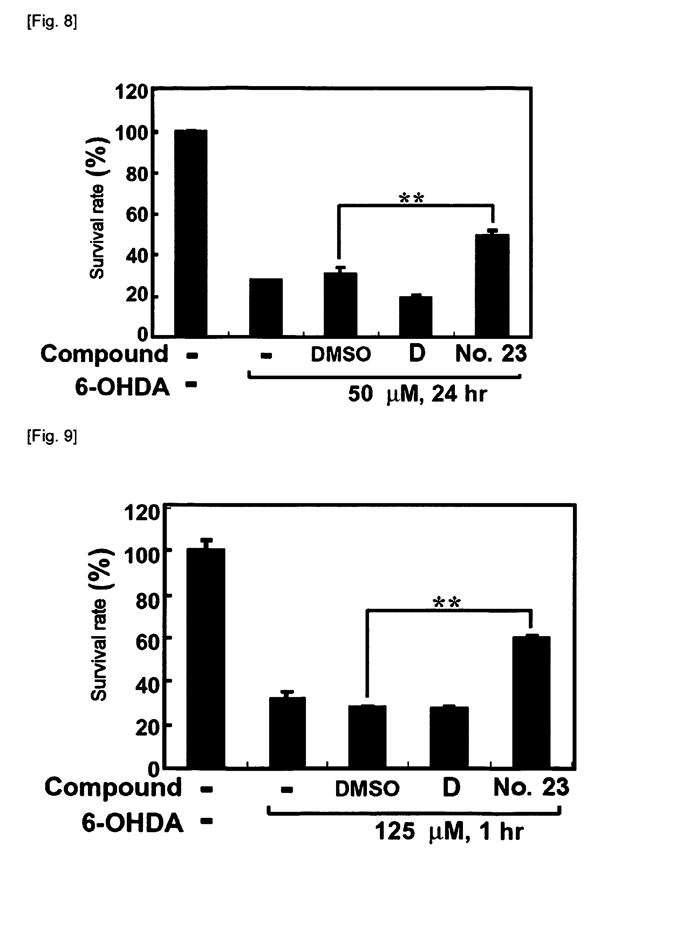

To a culture solution of human dopamine-producing cell SH-SY5Y, 1 µM DJ-1 bound compound (No. 23) dissolved in 0.01% DMSO was administered for 20 h, then the cultured solution was treated with 50 µM 6-OHDA (6-hydroxydopamine) for 24 h or with 125 µM 6-OHDA for 1 h; and the ratio of cell death was investigated by MTT assay. For comparison, a control (-) without addition of the DJ-1 bound compound and a control (DMSO) with addition of only DMSO, and a sample with addition of dopamine were used and subjected to the same method. Results are shown in Figs. 8 and 9.
In the sample (D) comprising dopamine, known as a therapeutic agent of Parkinson's disease, it was observed that No. 23 significantly suppresses the cell death. In addition, the administration of No. 23 did not exert toxicity for cultured cells.

### [Example 5] Active oxygen species-suppressive effect by DJ-1 bound compound

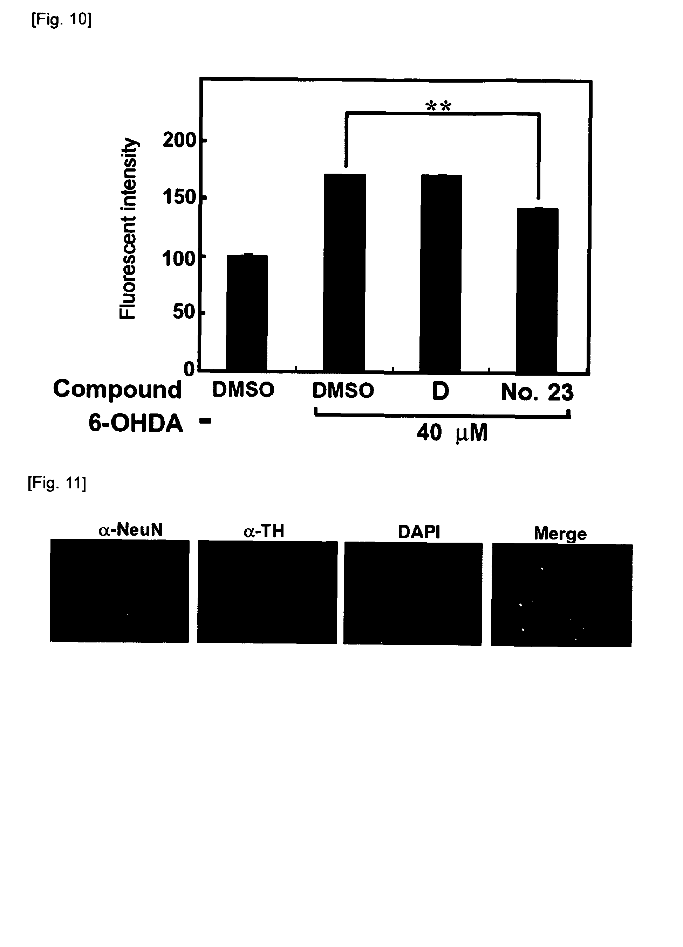

Human dopamine-producing cell SH-SY5Y was pre-treated with 1 µM DJ-1 bound compound (No. 23) for 20 h, and treated with fluorescent probe DCFA-DA, then exposed to 40µm 6-OHDA for 10 min. Subsequently, the active oxygen species was measured by a fluorescent spectrophotometer. For comparison, a control (-) without addition of the DJ-1 bound compound and a control (DMSO) with addition of only DMSO, and a sample with addition of dopamine were used and subjected to the same method. As shown in Fig. 10, it was observed that compared to dopamine, No. 23 significantly suppresses the production of active oxygen species caused by 6-OHDA.

### [Example 7] Nerve cell death-suppressive effect by DJ-1 bound compound

From germinal cells of a rat at 17-19 days pregnant, primary nerve cells of the rat ventral midbrain were prepared. To evaluate presence of dopamine-producing neurons in a cell culture, the cell culture was immunostained with anti-NeuN antibody and anti-TH antibody to identify each of whole neurons and dopamine-producing neurons, and cell nuclei were stained with DAPI. As shown in Fig. 11, almost all neurons were confirmed to be dopamine-producing neurons.

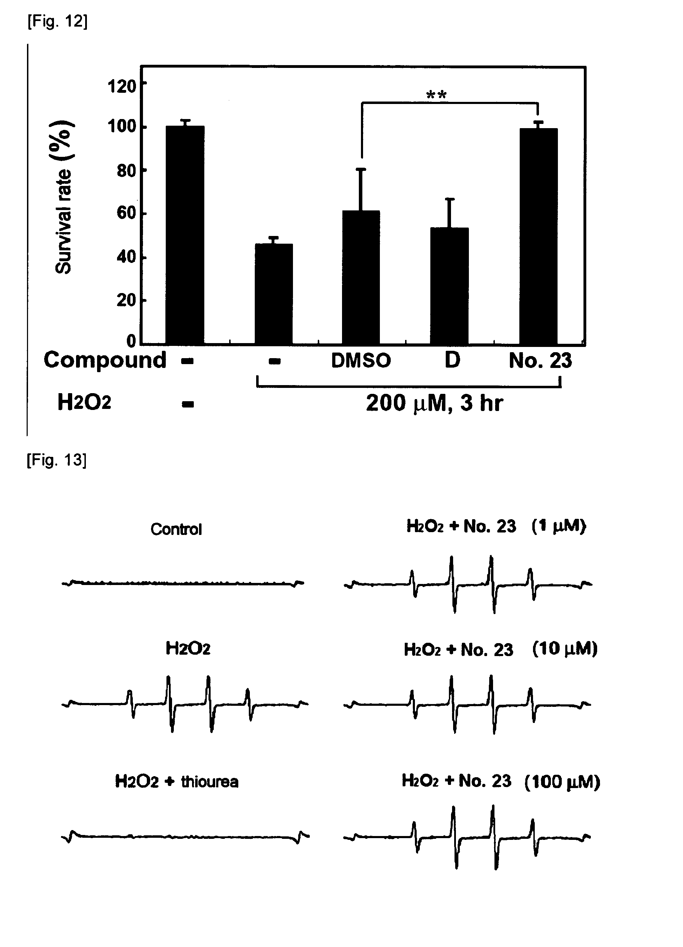

Next, the primary nerve cells were pre-treated with 1 µM DJ-1 bound compound (No. 23) dissolved in 0.01% DMSO for 20 h, then treated with 200 µM H₂O₂ for 3 h, and the ratio of cell death was evaluated by MTT assay. For comparison, a control (-) without addition of the DJ-1 bound compound and a control (DMSO) with addition of only 0.01% DMSO, and a sample with addition of dopamine were used and subjected to evaluation by the same method. As shown in Fig. 12, no cell death was observed in the sample in which No. 23 was administered. Therefore, it was clarified that No. 23 provides a resistance against nerve cell death induced by oxidative stress.

### [Example 8] Hydroxyl radical (·OH) -elimination ability of DJ-1 bound compound

Recent studies exhibited that H₂O₂ is generated by mitochondrial dysfunction or by auto-oxidation of dopamine and 6-OHDA, and hydroxyl radicals are easily generated from this H₂O₂ due to the presence of Fe²⁺. Hydroxyl radical is the most important neurotoxicity factor in dopamine-induced neurodegeneration. Therefore, to investigate whether a DJ-1 bound compound directly acts on hydroxyl radicals, evaluation was carried out by an electron spin resonance (ESR)-spin trapping method using 5,5-dimethylpyroline-N-oxide (DMPO).
To 200 µL of a 100-mM phosphate buffer reaction mixture containing 25 µM diethylene-triamine pentaacetic acid, 25 µM FeSO₄, 100 µM H₂O₂, and 112.5 mM DMSO, the 1 µM, 10 µM, or 100 µM DJ-1 compound (No. 23) and 500-mM thiourea were added respectively. For comparison, a phosphate buffer reaction mixture containing diethylene-triamine pentaacetic acid, FeSO₄, and DMSO, as well as the phosphate buffer reaction mixture further containing H₂O₂ were prepared. These drugs and reagents were dissolved in mili-Q water. The reaction mixture was transferred to a flat quartz cuvette, and introduced in X-band JEOL RFR-30b radical analysis system. Hydroxyl radicals generated from Fenton reaction between Fe²⁺ and H₂O₂ are trapped by DMPO. From 1 min after addition of DMPO, stable DMPO-OH adducts were measured. Signal of Mn²⁺ was used as the internal standard signal.

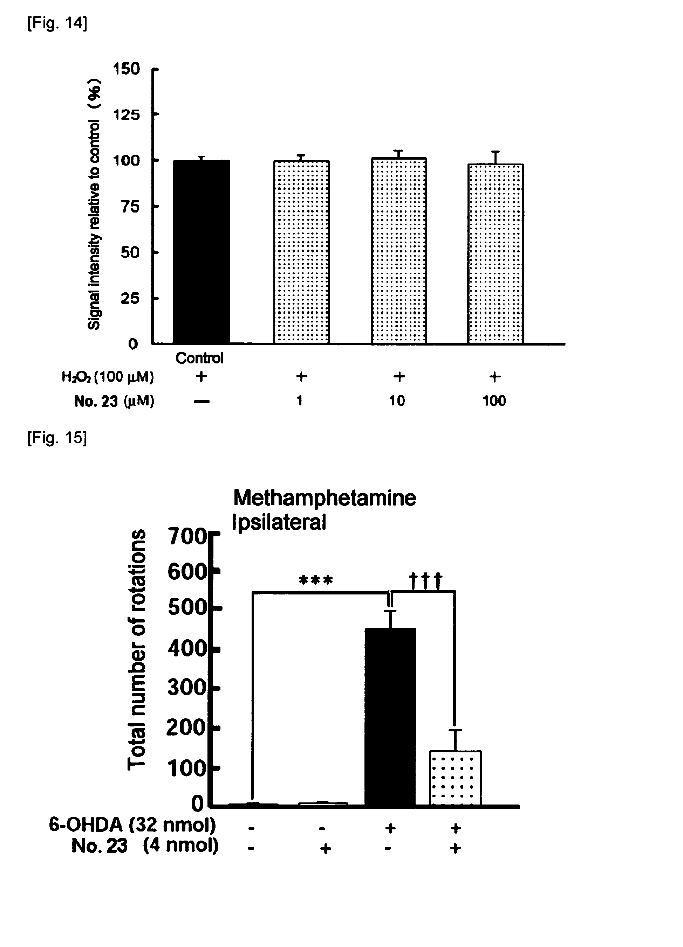

In the control without comprising H₂O₂ (Control) shown in Fig. 13, the peaks detected at both ends are signals of internal standard Mn²⁺, and other peaks are not detected because H₂O₂ is not present. In contrast, under the presence of Fe²⁺ and H₂O₂, peaks were detected with the intensity ratio of 1:2:2:1 between the two Mn²⁺ peaks (Fig. 13, see H₂O₂). These quadruple signals completely disappear by thiourea, a hydroxyl radical supplementing agent (Fig. 13, see H₂O₂ + thiourea), showing that the quadruple signals represent DMPO-OH adduct. In contrast to this, in the reaction mixture comprising No. 23, a decrease in peaks was not observed at a high concentration of even 100 µM (see Figs. 13 and 14). These suggest that No. 23 does not directly act to hydroxyl radicals, thus showing that No. 23 is not a merely anti-oxidation substance.

### [Example 9] Effects of DJ-1 bound compound on Parkinson's disease

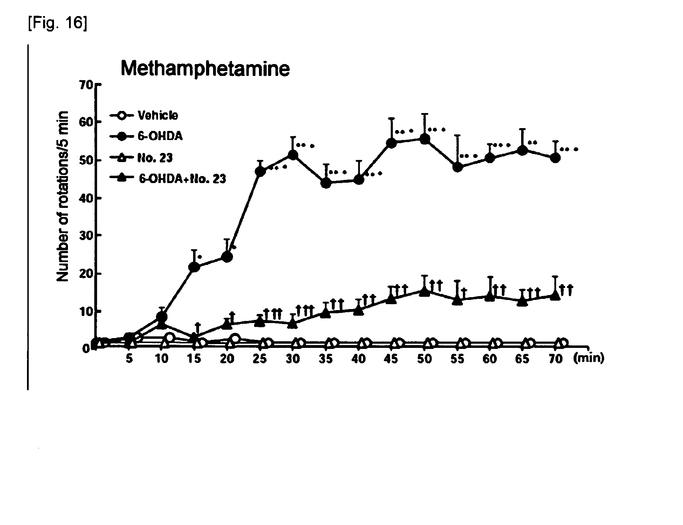

To evaluate effects of a DJ-1 bound compound on Parkinson's disease in vivo, 6-OHDA was stereotactically injected to the unilateral (left) midbrain of a mouse. Then, the DJ-1 bound compound (No. 23) was administered together with methamphetamine. Here, administration of methamphetamine to an animal induces ipsilateral movement relative to the injection site. Accordingly, by the administration of methamphetamine to a mouse, a rotational behavior is observed. Figure 15 shows the total number of rotational behaviors in mice up to 7 days after administration. Figure 16 shows time-course changes in the number of rotations. From Figs. 15 and 16, it is demonstrated that rotational behavior of mice is significantly suppressed by No. 23, and that No. 23 is extremely effective for the therapy of Parkinson's disease.

### [Industrial Applicability]

The neurodegenerative disease therapeutic agent of the present invention can highly suppress oxidative stress-induced nerve cell death, so that different from conventional symptomatic treatment, the agent of the invention enables more fundamental therapy for current neurodegenerative diseases by suppressing oxidative stress-induced nerve cell death.

## Claims

1. A therapeutic agent for a neurodegenerative disease comprising a compound represented by Formula (1): 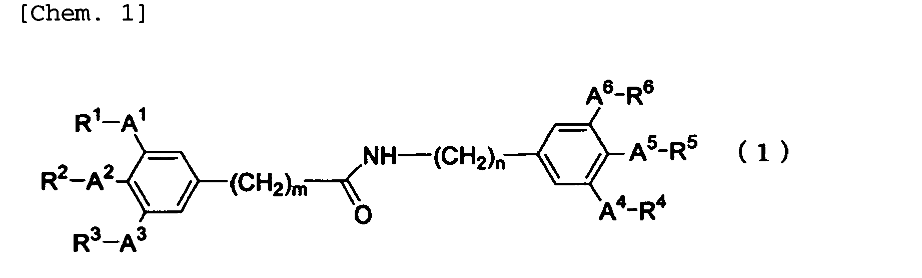 wherein
m is 0 or 1;
n is 0 or 1;
R¹ to R⁶ may be identical to or different from each other, and are a hydrogen atom, a hydroxyl group, a cyano group, a nitro group, a halogen atom, an amino group,
or
a straight or branched chain and saturated or unsaturated C₁₋₈ hydrocarbon which may have a substituent, a cycloalkyl group which may have a substituent, an aryl group which may have a substituent, a heterocyclyl group which may have a substituent;
A¹ to A⁶ are a single bond, -O-, -CO-, -COO-, -OCO-, -O-CO-O-, -S-, -SO-, -SO₂-, -O-SO₂-, -NH-, -NH-COO-, -NH-SO₂- and two adjacent -A¹-R¹ to -A⁶-R⁶ may form a ring together with the carbon atom to which they are attached;
or a pharmaceutically acceptable salt thereof.

2. The therapeutic agent for a neurodegenerative disease according to Claim 1, wherein m or n is 0.

3. The therapeutic agent for a neurodegenerative disease according to Claim 1 or 2, wherein m and n are 0.

4. The therapeutic agent for a neurodegenerative disease according to any one of Claims 1 to 3, wherein A¹ to A⁶ are a single bond, -O-, -CO-, -COO-, -OCO-, -O-CO-O-, -NH-, or -NH-COO-.

5. The therapeutic agent for a neurodegenerative disease according to any one of Claims 1 to 4, wherein R¹ to R⁶ may, each independently of one another, have a substituent, and are C₁₋₈ alkyl, C₂₋₈ alkenyl, C₂₋₈ alkynyl group, C₃₋₈ cycloalkyl, C₆₋₁₀ aryl, 5- to 10-membered heterocyclyl group comprising, in addition to a carbon atom, 1 to 4 heteroatoms selected from nitrogen atom, oxygen atom, and sulfur atom.

6. The therapeutic agent for a neurodegenerative disease according to any one of Claims 1 to 6, wherein at least one of -A¹-R¹ to -A⁶-R⁶ is a hydrogen atom, a hydroxyl group, or the following group which may have a substituent: a straight or branched chain C₁₋₈ alkyl group, a straight or branched chain C₁₋₈ alkoxy group, a straight or branched chain C₁₋₈ alkyl-carbonyl group, a straight or branched chain C₁₋₈ alkoxy-carbonyl group, a straight or branched chain C₁₋₈ alkyl-carbonyloxy group, a straight or branched chain C₁₋₈ alkoxy-carbonyloxy group, a C₆₋₁₀ aryl group, a 5- to 10-membered heterocyclyl group (comprising, in addition to a carbon atom, 1 to 4 heteroatoms selected from nitrogen atom, oxygen atom, and sulfur atom), a C₆₋₁₀ aryloxy group, a C₆₋₁₀ aryl carbonyl, a C₆₋₁₀ aryloxy-carbonyl group, a C₆₋₁₀ aryl-carbonyloxy group, a C₆₋₁₀ aryloxy-carbonyloxy group, a 5- to 10-membered heterocyclyloxy group, a 5- to 10-membered heterocyclyl-carbonyl, a 5- to 10-membered heterocyclyloxy-carbonyl group, a 5- to 10-membered heterocyclyl-carbonyloxy group, a 5- to 10-membered heterocyclyloxy-carbonyloxy group, a C₁₋₈ alkoxycarbonylamino group.

7. The therapeutic agent for a neurodegenerative disease according to any one of Claims 1 to 6, wherein -A²-R² and/or -A⁵-R⁵ are/is a hydrogen atom, a hydroxyl group, or the following group which may have a substituent: a straight or branched chain C₁₋₈ alkyl group, a straight or branched chain C₁₋₈ alkoxy group, a straight or branched chain C₁₋₈ alkyl-carbonyl group, a straight or branched chain C₁₋₈ alkoxy-carbonyl group, a straight or branched chain C₁₋₈ alkyl-carbonyloxy group, a straight or branched chain C₁₋₈ alkoxy-carbonyloxy group, a C₆₋₁₀ aryl group, a 5- to 10-membered heterocyclyl group (comprising, in addition to a carbon atom, 1 to 4 heteroatoms selected from nitrogen atom, oxygen atom, and sulfur atom), a C₆₋₁₀ aryloxy group, a C₆₋₁₀ aryl carbonyl, a C₆₋₁₀ aryloxy-carbonyl group, a C₆₋₁₀ aryl-carbonyloxy group, a C₆₋₁₀ aryloxy-carbonyloxy group, a 5- to 10-membered heterocyclyloxy group, a 5- to 10-membered heterocyclyl-carbonyl, a 5- to 10-membered heterocyclyloxy-carbonyl group, a 5- to 10-membered heterocyclyl-carbonyloxy group, a 5- to 10-membered heterocyclyloxy-carbonyloxy group, a C₁₋₈ alkoxycarbonylamino group.

8. The therapeutic agent for a neurodegenerative disease according to any one of Claims 1 to 7, wherein -A²-R² and/or -A⁵-R⁵ are/is a straight or branched chain C₁₋₄ alkyl which may have a substituent.

9. The therapeutic agent for a neurodegenerative disease according to any one of Claims 1 to 7, wherein -A²-R² and/or -A⁵-R⁵ are/is a monocyclic or bicyclic aryl group which may have a substituent.

10. The therapeutic agent for a neurodegenerative disease according to any one of Claims 1 to 7, wherein -A²-R² and/or -A⁵-R⁵ are/is a monocyclic or bicyclic aryl group substituted by a straight or branched chain C₁₋₄ alkyl.

11. The therapeutic agent for a neurodegenerative disease according to any one of Claims 1 to 7, wherein -A²-R² and/or -A⁵-R⁵ are/is a 5- to 10-membered monocyclic or bicyclic heterocyclyl group which may have a substituent and comprises, in addition to a carbon atom, 1 to 4 heteroatoms selected from nitrogen atom, oxygen atom, and sulfur atom.

12. The therapeutic agent for a neurodegenerative disease according to any one of Claims 1 to 7, wherein -A²-R² and/or -A⁵-R⁵ are/is a 5- to 10-memberedmonocyclic orbicyclic heterocyclyl group comprising, in addition to a carbon atom, 1 to 4 heteroatoms selected from nitrogen atom, oxygen atom, and sulfur atom, which is substituted by a straight or branched chain C₁₋₄ alkyl.

13. The therapeutic agent for a neurodegenerative disease according to any one of Claims 1 to 7, wherein at least one of -A¹-R¹ to -A⁶-R⁶ is a methoxy group.

14. The therapeutic agent for a neurodegenerative disease according to any one of Claims 1 to 6, wherein -A¹-R¹ to -A³-R³ are a methoxy group.

15. The therapeutic agent for a neurodegenerative disease according to Claim 1, wherein the two adjacent -A¹-R¹ to -A⁶-R⁶ form a ring together with the carbon atom to which they are attached, and said ring is a ring structure represented by Formula (2):

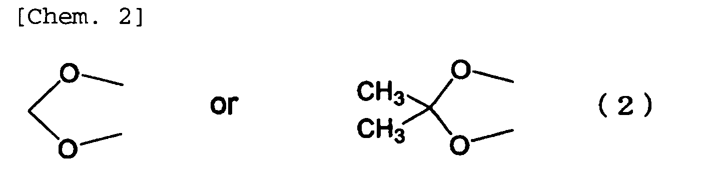

16. The therapeutic agent for a neurodegenerative disease according to Claim 1, wherein -A¹-R¹ and -A²-R² and/or -A⁵-R⁵ and -A⁶-R⁶ form a ring together with the carbon atom to which they are attached, and said ring is a ring structure represented by Formula (2):

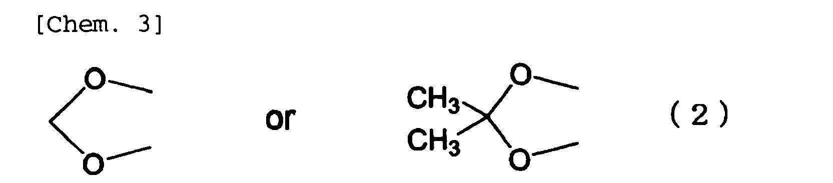

17. The therapeutic agent for a neurodegenerative disease according to Claim 1, wherein the compound represented by Formula (1) according to Claim 1 is a compound represented by Formulae (3) to (13):

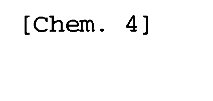

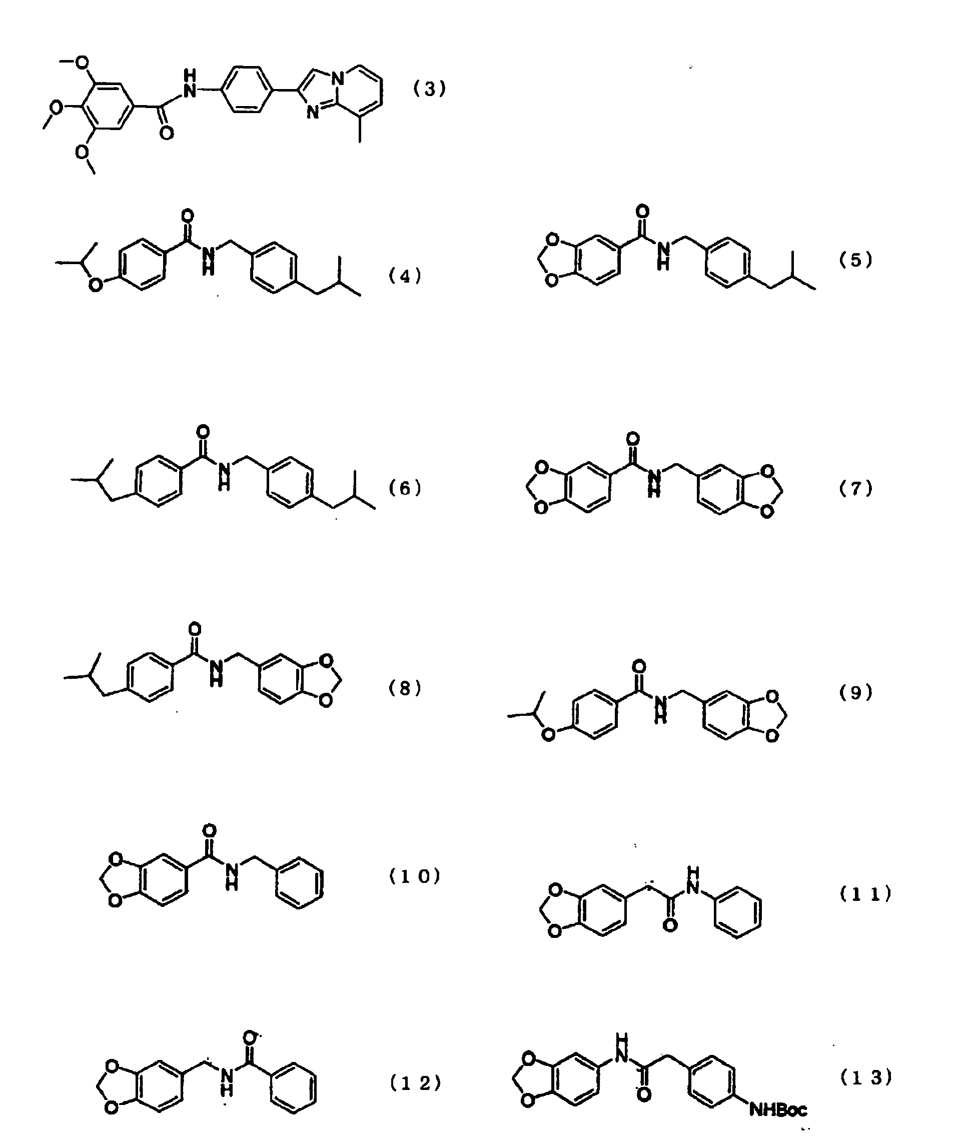

18. The therapeutic agent for a neurodegenerative disease according to Claim 1, wherein the compound represented by Formula (1) according to Claim 1 is a compound represented by Formula (3) :
